Europäisches Patentamt

European Patent Office

Office européen des brevets

(11) Numéro de publication: **0 312 452**
A1

# (12) DEMANDE DE BREVET EUROPEEN

(21) Numéro de dépôt: 88402582.6

(22) Date de dépôt: **12.10.88**

(51) Int. Cl.⁴: **A 61 N 1/16**
H 05 F 7/00, A 23 L 3/26,
C 12 H 1/16

(30) Priorité: **15.10.87 FR 8714256**

(43) Date de publication de la demande:
**19.04.89 Bulletin 89/16**

(84) Etats contractants désignés:
**CH ES GB GR IT LI**

(71) Demandeur: **Decup, Robert Claude**
**Résidence la Sirène 10, rue Henri Regnault**
**F-92400 Courbevoie (FR)**

**Antoine, Robert Julien Augustin**
**34, rue Marcel Robin**
**F-58640 Varennes Vauzelles (FR)**

(72) Inventeur: **Antoine, Robert Julien Augustin**
**34, rue Marcel Robin**
**F-58640 Varennes Vauzelles (FR)**

(74) Mandataire: **Derambure, Christian et al**
**BUGNION ASSOCIES 55, rue Boissonade**
**F-75014 Paris (FR)**

(54) **Générateur d'ondes.**

(57) La présente invention concerne un générateur d'ondes. De tels générateurs d'ondes peuvent principalement être utilisés pour les mises en oeuvre de méthodes naturelles bien connues de rééquilibrage syntonique des cellules vivantes, de revitalisation et, ou, de traitements des individus, personnes, animaux, plantes ou même, liquide ou objets inertes. Les produits résultant pourront ensuite être utilisés aux mêmes fins de syntonisation, soit par absorption, soit par rapprochement et, ou, contact, qui déclencheront automatiquement selon les lois attachées aux phénomènes de mise en résonance vibratoire, des syntonisations harmoniques des fréquences.

Ce générateur comporte : au moins un résonateur capteur (1-12), au moins un canon émetteur (13,14) associé à un ou plusieurs résonateurs capteurs (1-2) et amplifiant les ondes, un corps creux principal (15) destiné à syntoniser les ondes avec un corps (16) placé sensiblement en son milieu sur un support ou une stèle (17) vers lequel sont dirigées les ondes sortant des canons émetteurs.

FIG.1

EP 0 312 452 A1

# Description

## GENERATEUR D'ONDES

La présente invention concerne un générateur d'ondes. De tels générateurs d'ondes peuvent principalement être utilisés pour les mises en oeuvre de méthodes naturelles bien connues de rééquilibrage syntonique des cellules vivantes, de revitalisation et, ou, de traitements des individus, personnes, animaux, plantes ou même, liquide ou objets inertes. Les produits résultant pourront ensuite être utilisés aux mêmes fins de syntonisation, soit par absorption, soit par rapprochement et, ou, contact, qui déclencheront automatiquement selon les lois attachées aux phénomènes de mise en résonance vibratoire, des syntonisations harmoniques des fréquences.

Sans aucun apport extérieur d'énergie électrique et, ou électromagnétique, additionnelle et complémentaire quelle qu'elle soit de telles méthodes et, ou, de tels traitements rigoureusement naturels et, strictement sans danger, permettent d'obtenir une revitalisation, une stimulation, une tonification, une reconstitution et une recharge rapide d'énergie, doublée d'un étonnant ralentissement du vieillissement cellulaire basé, entre autre, sur l'élévation du taux vibratoire général des cellules par syntonisation.

Grâce à la mécanique ondulatoire, il est en effet prouvé aujourd'hui:

. Premièrement, que tout électron animé d'un mouvement accéléré rayonne au loin une partie de son énergie sous la forme d'une onde électromagnétique, et que, plus précisément, si des fragments de matière contiennent des électrons susceptibles d'osciller harmonieusement autour d'une position d'équilibre avec une fréquence déterminée, ceux-ci émettront un rayonnement électromagnétique ayant même fréquence que leur mouvement.

Tout donc vibre dans l'Univers, que ce soit la matière dite "inerte" ou la matière vivante. Et tout ce qui vibre émet des ondes longitudinales (linéaires) et, ou, tourbillonnaires.

De ce qui précède et, selon les lois de mise en résonance, si deux objets, à fortiori deux cellules vivantes vibrent à la même fréquence, ou sur une fréquence harmonique, ils tendent automatiquement et instantanément à entrer en résonance, même à des distances parfois considérables.

. Deuxièmement, que la cellule vivante grâce aux chromosomes du noyau, est désormais considérée comme étant un microscopique circuit oscillant électrique ouvert, rappelant le circuit oscillant de Hertz, doué de de fait, de self induction et de capacité. Donc susceptible d'osciller et de résonner à une fréquence très élevée, dont la vibration est entretenue par l'énergie rayonnante des ondes "magnéto-telluro-cosmiques".

. Troisièmement, que l'état de vie, et/ou, les manifestations de vie, sont les résultantes des mouvements vibratoires des cellules composant les corps, associés à des phénomènes de résonances et à des champs magnétiques d'intégrations s'inscrivant dans toutes leurs manifestations à l'échelon cellulaire.

. Quatrièmement, que si l'harmonie des vibrations cellulaires assure des fonctions vitales normales, le déséquilibre de ces vibrations, pour des causes externes et, ou, internes, crée par contre pour le moins des états de stress qui peuvent aller jusqu'aux états morbides.

. Cinquièmement, qu'en conséquence, puisque la constitution atomique de l'être vivant est inséparable de la réception et de l'émission d'ondes ou de radiations, ce fait, d'ordre strictement scientifique, fournit la preuve irréfutable, que, dans certaines conditions, la matière vivante peut être entretenue, modifiée ou alors détruite, par des radiations émanant de vibrations appropriées.

Jusqu'à ce jour, aucun dispositif important et efficace n'utilisant aucune énergie électrique additionnelle et complémentaire n'a été mis au point pour rétablir et, rééduquer par une action harmonieuse et rigoureusement naturelle, le régime vibratoire des cellules de la matière dite "inerte" ou, à plus forte raison, de la matière vivante par effet de syntonisation.

Il existe pourtant déjà depuis de nombreuses années, des appareils capteurs et émetteurs qui fonctionnent grâce à l'énergie électrique et, ou, électromagnétique, donc toujours avec une source d'origine humaine d'énergie d'appoint invariablement de provenance électrique industrielle.

Mais, sans être entièrement néfastes et relativement dangereuses, les ondes ainsi captées puis émises par ces appareils, sont néanmoins, particulièrement difficilement assimilables par la presque totalité des individus.

Ces ondes sont en effet en grande partie, relativement dénaturées et, défavorablement modifiées par les interférences d'origine électrique qu'il est absolument impossible d'éviter, par le fait même qu'il y a apport d'énergie électrique pour faire fonctionner ces appareils.

C'est pour cette raison fondamentale, que ces procédés de régénération, et de revitalisation par effet de syntonisation, bien que découverts depuis fort longtemps, ont presque été mondialement abandonnés.

C'est l'objet de la présente invention de proposer des générateurs d'ondes efficaces qui permettent sans danger, mais surtout, sans aucun apport d'énergie électrique et, ou, électromagnétique d'appoint, d'obtenir entre autre , un étonnant ralentissement du vieillissement cellulaire, une recharge rapide d'énergie, une stimulation générale, une tonification particulière, une revitalisation spécifique, une reconstitution exceptionnelle et un rééquilibrage syntonique des cellules quelles qu'elles soient, par une élévation spontanée de leur taux vibratoire aux fréquences bien connues d'un parfait état de santé et d'un plus grand potentiel énergétique.

A cet effet, il est proposé, selon l'invention, un générateur d'ondes caractérisé en ce qu'il comporte :

- au moins un résonateur capteur,
- au moins un canon photonique émetteur d'électrons associé à un ou plusieurs résonateurs capteurs amplifiant les ondes,
- un corps creux principal destiné à syntoniser les ondes avec un corps placé sensiblement en son milieu sur un support ou une stèle vers lequel sont dirigées les ondes sortant des canons émetteurs d'électrons.

L'invention sera mieux comprise à la lecture de la description qui va suivre, faite en référence aux dessins dans lesquels :

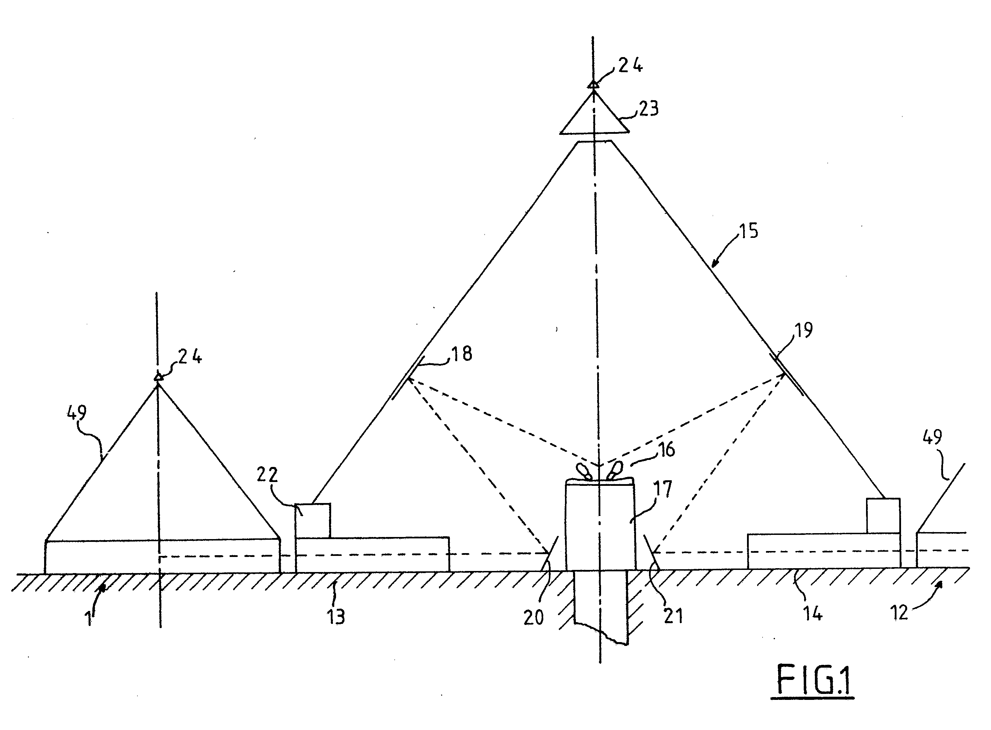

. La figure 1 représente une vue en coupe verticale d'un dispositif générateur d'ondes conforme à l'invention.

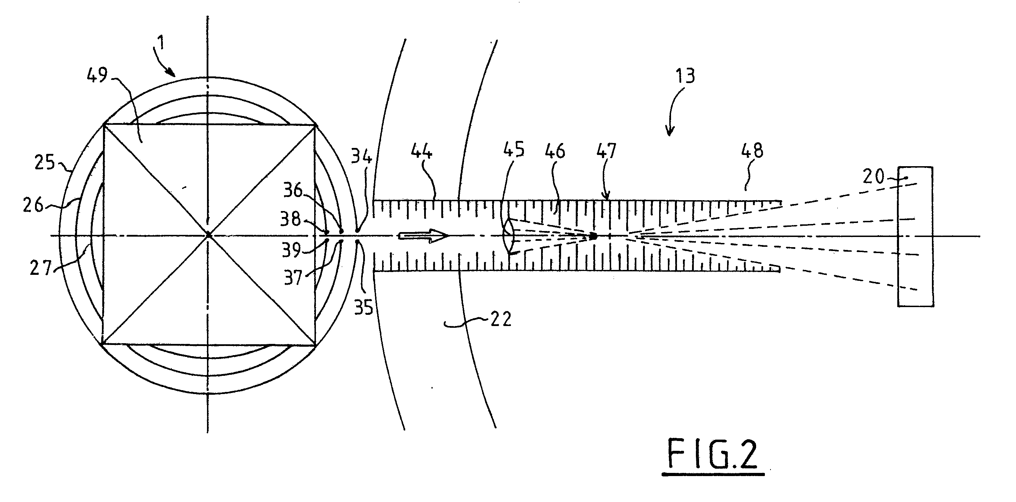

. La figure 2 représente une vue du dessus d'un ensemble résonateur capteur, canon photonique émetteur d'électrons selon un premier mode de réalisation.

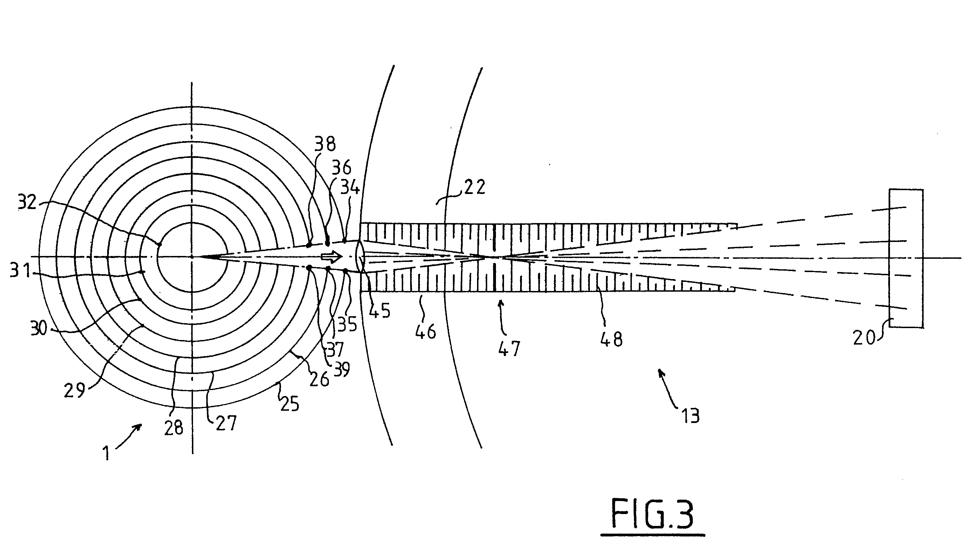

. La figure 3 représente une vue du dessus d'un ensemble résonateur capteur, canon photonique émetteur d'électrons selon un deuxième mode de réalisation.

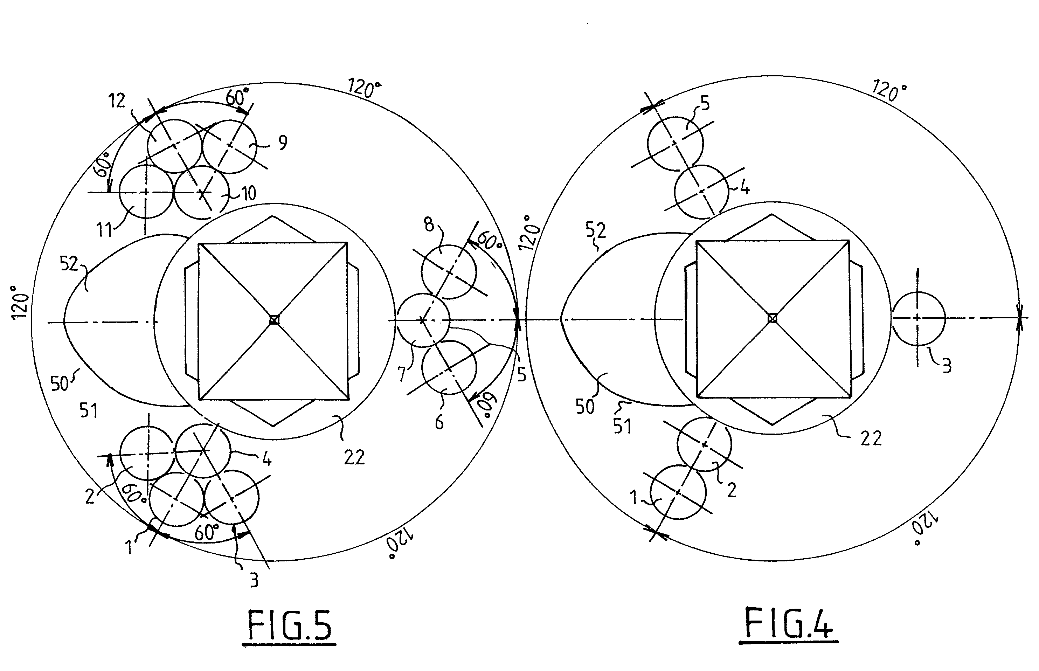

. Les figures 4 à 11 représentent différentes configurations de générateurs d'ondes conformes à l'invention et vues de dessus.

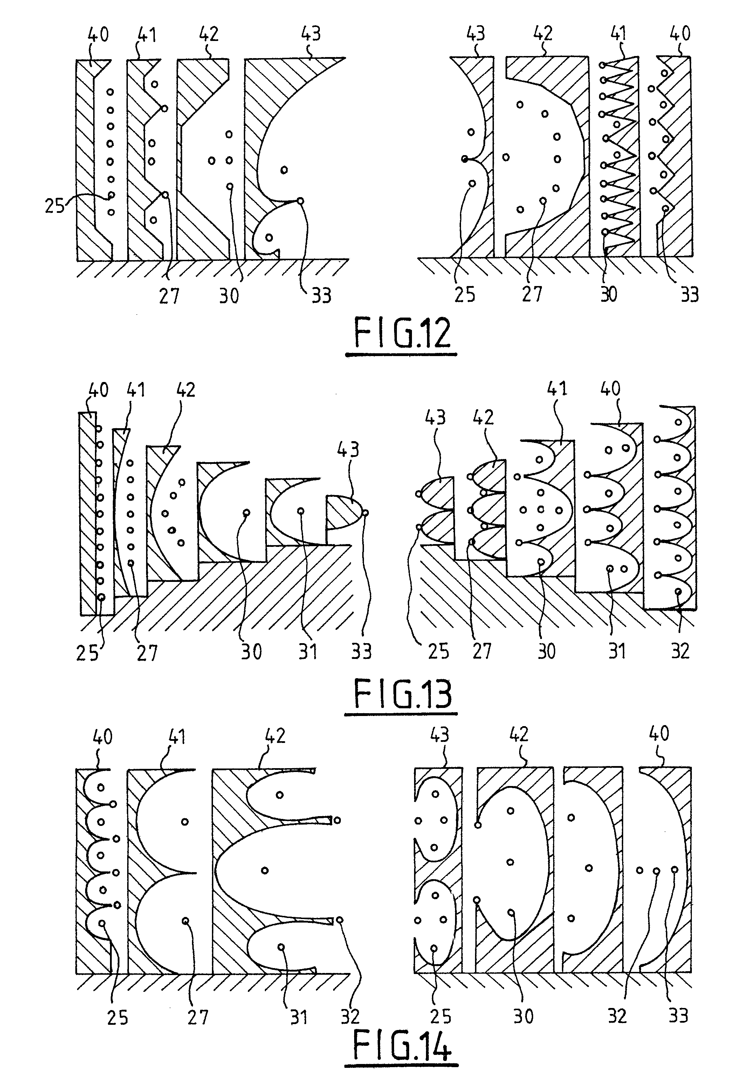

. Les figures 12, 13, 14 représentent des coupes verticales des structures émettrices d'effets de forme.

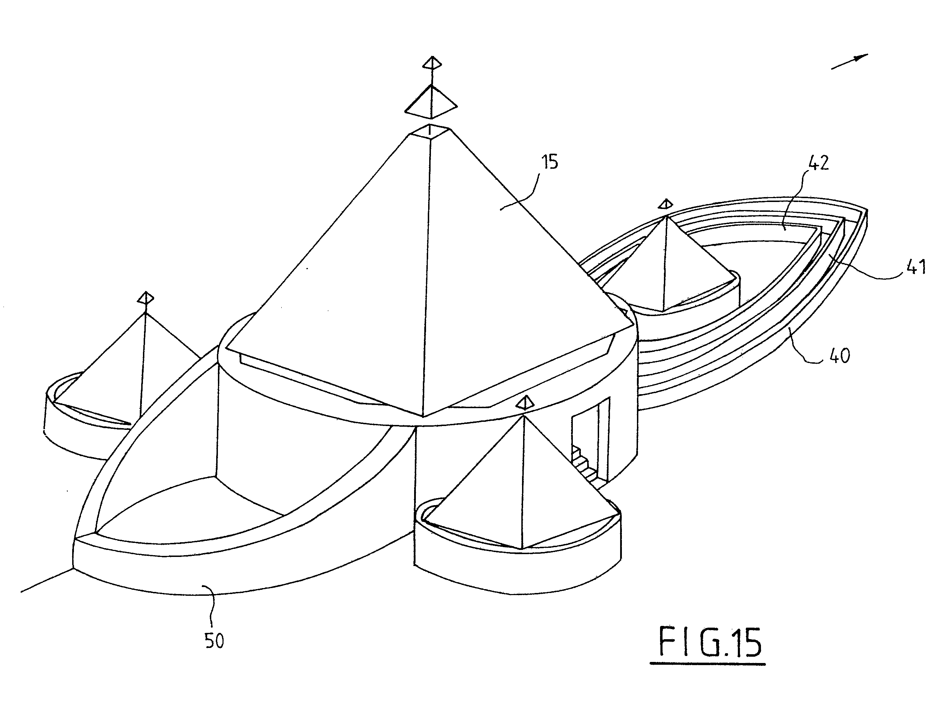

. La figure 15 représente une vue en perspective d'un des générateurs d'ondes selon l'invention, dans une grande dimension spécialement réalisée pour les syntonisations des cellules vivantes du corps humain.

L'origine de l'invention provient entre autre , comme il a été déjà précédemment longuement expliqué, de la constatation faite par un certain nombre de savants et de chercheurs du monde entier qui ont pu également le démontrer, que la vie, et même l'organisation de la matière sont fondées sur les vibrations.

Si certaines vibrations sont susceptibles de perturber, même très gravement, les oscillations cellulaires, par contre certaines ondes "magnéto-telluro-cosmiques" convenablement captées sans interférences électriques et, ou, électromagnétiques, donc uniquement par les actions des micro-courants induits dans les circuits oscillants des résonateurs statiques devant obligatoirement et, impérativement fonctionner par simple mise en résonance, peuvent, après avoir été parfaitement captées, exactement concentrées, correctement accélérées puis, convenablement amplifiées, agir excessivement favorablement sur les micro-oscillateurs que sont les cellules des organismes vivants et, sans doute, aussi sur les molécules de la matière dite "inerte".

Malgré le grand nombre de travaux développés jusqu'à ce jour, les expériences réalisées restent parcellaires et aucun dispositif fonctionnant rigoureusement sans aucun apport d'énergie électrique et, ou, électromagnétique d'appoint, n'a jusqu'à présent été proposé qui permette d'assurer des couplages harmoniques complets des dites ondes, avec les cellules des organismes vivants et, très certainement, avec les molécules de la matière dite "inerte".

Les générateurs d'ondes de l'invention produisent exactement ces résultats depuis si longtemps recherchés. Afin de produire de tels effets, le générateur d'ondes selon l'invention, comporte au moins un résonateur capteur (1-12). Le corps creux principal 15 est lui-même destiné à capter les ondes et à entrer en résonance. Cela lui permet ensuite de syntoniser l'ensemble des ondes captées ou produites avec le corps 16.

Les résonnateurs capteurs (1-12) peuvent être insérés dans une au moins des structures émettrices d'effets de formes (40-43).

Le fonctionnement de tels dispositifs est le suivant : il est connu que des ondes magnétiques, telluriques et cosmiques, favorables ou défavorables aux vibrations cellulaires existent à l'état naturel.

Il est connu aussi que ces ondes sont plus fournies et de plus grande intensité , qu'à de très rares endroits et, si l'homme à toujours voulu se préoccuper d'éviter les endroits trop perturbés, il est reconnu que malheureusement, l'intensité et aussi la densité des ondes bénéfiques des lieux très agréables, sont beaucoup trop faibles pour pouvoir produire des effets sensibles en un court espace de temps sur les cellules et, ou, les molécules qui les reçoivent.

Les dispositifs de l'invention, ont pour fonction de capter par simple mise en résonance harmonique et, sans aucun apport d'énergie électrique et, ou, électromagnétique, produite et fournie industriellement par l'Homme des ondes naturelles favorables puis, de les concentrer, de les amplifier, de les accélérer et d'en produire d'autres parfaitement harmoniques par le phénomène de la résonance. De plus, des dispositifs spécifiques d'une part et, les harmoniques bénéfiques produites d'autre part, arrêtent et, ou, éliminent les ondes défavorables.

A cet effet, les résonateurs capteurs 1-12, captent et produisent aux éclateurs 34 à 39, des circuits oscillants polymétalliques 25 à 33, les ondes électromagnétiques des micro-courants induits provenant des rayonnements électromagnétiques ambiants, qui sont ensuite concentrées, amplifiées, accélérées et enfin focalisées par le ou les canons photoniques émetteurs d'électrons 13-14.

En physique classique, il est bien connu que tous circuits oscillants simples, assimilés à des "dipôles", s'ils sont convenablement orientés, entrent automatiquement et instantanément en résonance, en devenant le siège de courants électriques induits par les variations des champs magnétiques ambiants.

Ces champs magnétiques ambiants sont composés de l'électricité naturelle atmosphérique, du champ magnétique terrestre et, de tous ceux créés par les courants alternatifs qui apparaissent dans les circuits par les effets des ondes électromagnétiques des stations de radio, télévision, radars militaires ou civils de protection, etc...

On sait que la tension aux extrémités d'un tel anneau ouvert ou "dipôle", est donnée par la relation :

$$u = E \times \frac{\lambda}{\pi},$$

U, étant exprimé en millivolts, E (le champ électromagnétique ambiant) en millivolts/mètre et $\lambda$ en mètres.

Et l'on sait aussi, que le champ électromagnétique ambiant créé par un émetteur de puissance P, situé à la distance D, est donné par la relation :

$$E = \frac{300V \cdot P}{D}$$

dans laquelle, E est exprimé en millivolts/mètre, P en kilowatts et D en kilomètres.

On en conclut que sous le seul et unique effet des émissions de radiocommunications hautes fréquences (télévisions, radars, etc...) une tension non négligeable apparaît aux bornes des de ces circuits oscillants sans que l'on fasse appel à une source d'énergie additionnelle.

A titre indicatif, le gradient moyen de tension recueillie aux bornes d'une simple antenne de télévision convenablement orientée sur un toit, peut atteindre les 300 millivolts à certains endroits. Ainsi avec plusieurs antennes montées en série, il est réellement possible de faire fonctionner un petit poste de radio à transistors, sans autre source d'énergie.

Ces micro-courants induits naturellement dans les circuits oscillants 25 à 33, dans les résonateurs capteurs 1-12 qui couvrent un spectre radioélectrique théoriquement infini, ainsi que dans les différents étages et ampilages des condensateurs accélérateurs 44-46-48, sont ensuite amplifiés et accélérés grâce, justement, à ces multiples ampilages et étages qui, de plus, émettent également à leur très nombreux éclateurs 34 à 39 et à l'infinité des pointes des innombrables éléments des condensateurs accélérateurs 44-46-48, automatiquement et spontanément des électrons.

Lesquels se combinent aux constituants normaux de l'air, ce qui contribue à former des "ions négatifs", des "ions métalliques" et, plus particulièrement, à produire une "ionisation négative" de l'air atmosphérique.

Les canons photoniques émetteurs d'électrons 13-14 à leur mise en marche immédiate, dès le montage terminé par entrée instantanée en résonance, produisent la formation d'un nuage d'électrons comme précédemment expliqué et créent de puissants champs accélérateurs pour ces électrons.

Ces électrons atteignent alors, grâce aux différents et successifs étages 44-46-48 d'accélérateurs et amplificateurs de très grandes vitesses.

Très grandes vitesses qui leur confèrent mathématiquement, une plus grande énergie qui va permettre d'exciter encore plus, le milieu ambiant du point de concentration maximum au foyer 47, ainsi qu'au point focal général 16 sur la stèle 17.

Les molécules de ce mélange ionique, vont donc atteindre un niveau d'énergie forcément supérieur à leur potentiel initial d'ionisation.

Comme toujours à cet instant, les atomes sont alors impérativement l'objet de restructurations forcées et obligatoires, qui les conduisent nécessairement à éjecter des électrons lorsque ces molécules reviennent à leur niveau fondamental.

De ce fait, il est évident qu'il y a une forte émission de photons de différentes énergies, dont les plus puissants, ioniseront encore plus, d'autres molécules du milieu ambiant du foyer 45 ainsi qu'au point focal général 16 sur la stèle 17.

Comme la physique moderne enseigne que l'échange d'énergie se fait par échange de particules, et qu'elle enseigne aussi que la force électromagnétique est véhiculée par les photons, l'interaction électromagnétique des ondes électromagnétiques des micro-courants induits dans l'ensemble des Générateurs d'Ondes selon l'invention et, comme précédemment expliqué, se fait par échange de photons.

Perpétuellement et automatiquement auto-alimentés par simple induction, ce qui garantit une innocuité absolue par l'auto-régulation et l'auto-stabilisation, les canons photoniques émetteurs d'électrons 13-14, à très faible puissance rayonnée, verront d'ailleurs relativement facilement, l'exactitude de la cinématique de leur fonctionnement, par ailleurs bien connue en physique, prouvée et, démontrée par l'emploi de la l'électrographie, la spectroscopie ou de l'électroscopie, qui permettront des éclatantes mises en évidence de très importants "Effets Kirlian" que les soviétiques nomment "BIOPLASMA" observés par les variations des "bioluminescences" et que la science et la physique modernes considèrent comme étant le cinquième état de la matière.

Ces effets et bioluminescences n'existant plus dès que les divers appareils sont même sommairement déréglés par exemple en modifiant tout simplement les orientations des multiples éléments.

De même, cette brillante mise en évidence, pourra être doublée et également magistralement confirmée par les spectrogrammes d'un Spectrophotomètre "RAMANLASER", d'une eau simple par exemple, qui sera devenue très exceptionnellement enrichie d'une énorme quantité de molécules "Trimères" universellement reconnues comme étant les plus actives au niveau biologique, tout simplement après avoir séjournée même un court instant au point de concentration général maximum 16 sur la stèle 17.

Les faisceaux d'ondes alors transmis vers le corps creux principal 15, lui-même capteur d'ondes de longueurs obligatoirement différentes puisque, entre autre , n'entrant pas en résonance de par sa masse, ses formes et ses matériaux, de la même façon que les résonateurs capteurs 1 - 2, sont correctement acheminés puis, plus spécifiquement pour les générateurs d'ondes devant modifier bénéfiquement par syntonisation la fréquence vibratoire des cellules humaines vivantes, réparties par l'intermédiaire des miroirs 18 à 21, sur tout corps 16 placé au centre du corps creux 15, par exemple sur une stèle 17, de façon à produire par syntonisation, les effets bienfaisants ainsi obtenus.

La synthonisation qui permet par l'accord de mise en résonance des ondes captées et créées à une fréquence permettant d'augmenter les effets bénéfiques, de neutraliser les effets néfastes et de modifier favorablement la fréquence vibratoire d'un individu, d'un objet ou aussi, d'un environnement, est l'un des éléments essentiels de l'invention.

Suivant les buts recherchés, les intensités d'émissions désirées et, les effets d'ensembles souhaités, le corps creux principal 15, bien qu'étant préféré de forme pyramidale, ceci principalement pour les syntonisations des cellules vivantes, pourra être très avantageusement et, dans certains cas, remplacé par un volume de résonance cubique, cylindrique, , parallélépipédique régulier ou quelconque, conique ou pyramidale mais à base polygonale ayant un nombre de faces réduit quelconque, ou, bien au contraire, très important et régulier.

Les expériences de recherches l'ayant prouvé, et même, la chose en soi ne devant pas surprendre dans le domaine des phénomènes encore bien peu connu des émissions dues aux formes, par deux volumes de formes définies comme précédemment, opposés par exemple soit par leurs pointes pour des formes coniques ou pyramidales, soit, après ce premier montage réalisé, par leurs plus grandes bases pour les étages supérieurs successifs.

De tels montages peuvent être alors appelés : "PILES EMETTRICES". En effet, le nombre des éléments ainsi superposés, pourrait selon une frappante similitude empruntée aux lois électriques, désigner le voltage et, ou l'importance, et les masses mises en jeux pourraient exprimer l'ampérage.

Ces constructions plus spécifiquement réservées aux influences non biologiques, quoique les efficacités soient particulièrement étonnantes et considérables sur les végétaux, ont donné d'excellents résultats pour modifier par syntonisation et, ou, ébranlement vibratoire (ébranlement vibratoire qui n'ont toujours pu être exactement mesuré faute d'appareils assez sensibles) des liquides comme par exemple des boissons, des nourritures et des minéraux.

Toutes les actions, influences, effets et, ou, modifications par ces actions, influences et effets, ont pu être démontrés par les observations et les résultats d'expériences.

Par exemple, certains vins et certains alcools, après avoir été soumis bien peu de temps (qui est toujours variable suivant les provenances) aux influences de tels générateurs d'ondes selon l'invention, ont acquis des qualités dégustatives qui ne peuvent être obtenues que par de très longs vieillissements.

Certaines boissons toniques, et certains mets forts simples, même après des temps très courts d'expositions aux effets, actions et influences de tels générateurs d'ondes, sont devenus sans n'être qu'une simple apparence, beaucoup plus stimulants et particulièrement beaucoup plus reconstituants.

Les ébranlements vibratoires particulièrement bienfaisants ainsi obtenus par certains générateurs d'ondes selon l'invention qui ont été modulés en conséquence à leurs constructions et réalisations pour obtenir des actions, influences et effets spécifiques sur les boissons, les nourritures, les végétaux, les minéraux et les liquides, comme par exemple, l'eau d'arrosage pour les cultures, sont également un autre élément essentiel de l'invention.

Pour un emploi principalement réservé en biologie, considérée ici dans l'acceptation la plus large, et selon un mode de réalisation préféré, le corps creux principal 15 est pyramidal.

En effet, l'expérience a démontré que cette forme est celle qui produit le syntonie cellulaire qui convient le mieux aux êtres vivants, et qui est relativement la mieux supportée en général.

Selon un mode de réalisation préféré, les parois de cette pyramide sont très avantageusement confectionnées à l'aide de multimatériaux comme, les métaux magnétiques ou non, associés en panneaux sandwichs avec des matériaux organiques et, ou, minéraux diélectriques ou non.

Selon un mode de réalisation préféré, les proportions de cette pyramide sont identiques à celles de la grande pyramide d'Egypte dite de CHEOPS, dans laquelle le périmètre de base est égal à la circonférence ayant la hauteur comme rayon.

Pour avoir une efficacité maximum, l'une de ses faces est orientée vers le Nord magnétique. Par contre, s'il est recherché une action beaucoup plus douce, c'est une arête de la pyramide qui devra être orientée au Nord magnétique.

Ainsi, les techniques actuelles de construction mécanique, permettent de réaliser relativement facilement, des générateurs d'ondes selon l'invention, qui pourraient avantageusement combiner les deux extrêmes de ces effets, en étant tout simplement montés sur un berceau circulaire pouvant même être orienté sur 360°, permettant ainsi au patient désirant bénéficier par exemple, d'une revitalisation plus ou moins forte, de moduler lui-même, par simple orientation de l'ensemble, la puissance d'émission d'un tel générateur d'ondes.

Ainsi, l'un au moins des corps creux est porté par un support sensiblement horizontal et monté tournant autour d'un axe vertical.

Selon un mode de réalisation préférée, le corps creux principal 15 ne repose pas directement sur le sol. Il repose sur une base en simple maçonnerie sans armature métallique. Cette solution étant considérée comme relativement meilleure. Mais cette base pourra être, suivant les buts recherchés et, ou, l'intensité d'émission souhaitée, en d'autres matériaux comme les métaux, ou, par exemple, les matériaux de synthèse et, ou, les matériaux composites.

Cette base qui dans un mode préféré de construction est cylindrique extérieurement et polygonale intérieurement, pourra, suivant les émissions et les effets de forme réclamés, être également polygonale extérieurement avec, ou sans portions cylindriques combinées, et, comporter également, les mêmes configurations intérieures.

Etant entendu et bien compris, que grâce à toutes les combinaisons possibles, les intensités et les étendues des émissions et des effets de forme sont ainsi obtenues, et que de plus, par les orientations au Nord magnétique soit d'un angle, soit d'un côté de ces polygones, toutes ces émissions et tous ces effets de forme, sont modulables à l'infini.

L'expérience a montré que le captage initial, l'amplification ensuite, et enfin l'harmonisation parfaite de la syntonisation des ondes dans le corps creux de résonance 15, sont réalisés d'une façon particulièrement eurythmique, lorsque la pointe de cette pyramide est tronquée dans un rapport assez vaste allant du dixième au trentième de la hauteur. L'ampleur de ce rapport ne semble pas modifier, d'une façon sensible, les gains acquis.

D'une manière analogue, il a également été constaté que ces gains supplémentaires, sont encore amplifiés, lorsque le corps principal de résonance 15, quelqu'il soit, est surmonté d'un deuxième corps de résonance 23, qui cette fois peut être plein ou toujours creux si nécessaire.

Ce deuxième corps doit être, de préférence, homothétique du premier, dans un rapport non limitatif qui est avantageusement de 1 sur 10.

De plus, il y a avantage à munir la base de ce deuxième corps de résonance, d'un miroir dont la face réfléchissante doit se trouver en regard du premier corps creux principal.

Par économie et, ou, pour une simplification de construction, ce miroir peut être tout simplement plan, mais il est relativement profitable, de choisir suivant les effets désirés, soit un miroir diffuseur convexe, soit un miroir concentrateur concave de plus grande efficience.

Tous ces effets sont encore augmentés et renforcés, lorsque ce deuxième corps de résonance 23, est lui-même, surmonté d'un troisième corps homothétique du second, qui est, lui aussi, et non limitativement réalisé dans un nouveau rapport de I sur 10. Troisième corps homothétique, qui cette fois n'a plus la nécessité d'être creux, mais qui, bien entendu peut l'être.

D'une manière surprenante, l'expérience a démontré que le fonctionnement de l'appareil est meilleur lorsque ce troisième corps est en cristal pur.

Enfin, d'une manière aussi surprenante, l'expérience a démontré qu'un fonctionnement presque parfait du système est obtenu lorsqu'un puits équilibreur 55 de diamètre et de profondeur eurythmique avec le corps creux de résonance 15 ou un volume de même importance est creusé dans le sol sous le support, ou, la stèle 17 juste à l'aplomb des corps 23 et 24.

Selon un mode de réalisation préféré, les résonateurs capteurs 1-12, en dehors de leurs émissions et effets de forme respectifs, comportent des circuits oscillants métalliques et, ou, polymétalliques 25 à 23 judicieusement disposés sur des murets supports conformément aux représentations des figures 12-13 et 14, chacun comprenant des circuits conducteurs plan quasi fermés terminés à leurs extrémités par des éclateurs 34 à 39.

Les résonateurs capteurs peuvent comporter plusieurs circuits contenus dans des plans parallèles et de forme polygonale régulière ou circulaire.

Calculé pour émettre des longueurs d'ondes qui soient en parfaite syntonie avec les corps qui doivent bénéficier de leurs bienfaits, chacun de ces circuits conducteurs, polymétalliques ou non, représente à lui seul, un circuit oscillant.

Chacun de ces circuits oscillants 25-33 peut être réalisé soit, pour plus de facilité de construction, dans un métal donné, soit beaucoup plus avantageusement, en combinaison ou non, avec le premier montage, et, ou, pour chaque module d'émissions et d'effets de forme, par un faisceau torsadé ou non, de nombreux fils et ou, câbles de métaux absolument différents.

Métaux différents qui dégagent un rayonnement spécifique qui est en fonction du nombre atomique de ce corps métallique. Ainsi le Fer par exemple, qui possède le numéro atomique 26, émet un rayonnement correspondant à ses 26 protons et électrons, l'Or qui a le numéro atomique 79 émet un rayonnement correspondant à ses 79 protons et électrons, etc...

Emis, accélérés puis projetés par les actions de bombardements et de transferts dynamiques des canons photoniques émetteurs d'électrons 13-14, ces ions négatifs et ces ions métalliques, sont acheminés et guidés grâce aux miroirs 18-19-20-21 au point de concentration général maximum 16 sur la stèle 17 ou, par effet d'osmose, ils sont très facilement absorbés par la peau des êtres désirant en bénéficier, ainsi qu'inhalés par leurs respirations. Ceux-ci baignent en quelque sorte, dans un véritable nuage nourrissant.

Bien qu'étant en fait, d'origine électrique pure, les micro-courants, induits naturellement par induction magnétique, ne sont absolument pas néfastes comme l'est par exemple, un apport extérieur d'énergie électrique d'origine humaine, bien au contraire, puisque étant de par leur naissance, automatiquement en parfaite syntonie avec l'ensemble du, ou des, générateurs d'ondes selon l'invention.

Les cations métalliques ainsi créés, forment avec diverses molécules organiques, des macro-molécules complexes dans lesquelles la partie métallique est essentielle au fonctionnement correct. Les métallo-enzymes catalysent en effet dans les organismes vivants, des réactions d'oxydoréduction, des transferts etc... qui sont absolument indispensables à la vie.

Ces métallo-enzymes syntonisent alors d'autant mieux avec l'organisme, ainsi baigné par les effluves des faisceaux d'ondes diffusés par les canons émetteurs 13-14, par le fait qu'à cet instant, les accords des fréquences générateur-organisme, sont absolument parfaits.

L'expérience ayant prouvé que les absorptions par inhalation et effet d'osmose, atteignent à ce moment, leurs intensités maximum. Cela contribue à apporter entre autre et en quantité suffisante, par amélioration de meilleure assimilation, les métaux nécessaires à l'organisme et à supprimer en partie, les carences dans ce domaine.

En effet, la liste des métaux jugés essentiels pour l'organisme s'allonge régulièrement, mais il est déjà admis que, si l'ensemble des métaux contenus dans le corps humain ne représente pas plus de 0,5 pour mille de la matière sèche, soit tout de même près de 3,5 g pour une personne du 70 kg, les besoins journaliers de l'organisme sont néanmoins compris entre, 18 à 20 mg pour le Fer, 2 à 3 mg pour le Cuivre et le Zinc, mais dépasse bien souvent les 300 mg pour le Magnésium.

L'expérience a prouvé que le choix judicieux des métaux devait être : l'Or, l'Argent, le Cuivre, le Zinc, le Fer, le Nikel, le Chrome, l'Etain, le Manganèse, l'Aluminium, le Vanadium, le Molybdène, le Cobalt, et bien entendu le Magnésium.

Les générateurs d'ondes selon l'invention, en dehors de la parfaite harmonisation des régimes vibratoires des cellules, communiquent donc aux organismes soumis à leurs bienfaisantes influences, d'une part, une certaine énergie spécifique capable de soulager, voir de guérir parfois, et, d'autre part, permet de faire un apport métallique qualitatif comparable à ceux réalisés en homéopathie, en oligothérapie, en thalassothérapie etc...

Pour obtenir ces incomparables résultats, toutes les dimensions des générateurs d'ondes selon l'invention, devront pour se trouver parfaitement en harmonie de syntonisation avec les ondes captées, être de préférence, calculées comme dans toutes les belles architectures, avec des proportions dites : de moyenne et extrême raisons qui définissent ce qui est appelé la Divine Harmonie, ou la Section Dorée ou encore, les Divines Proportions.

Le meilleur captage des ondes a été constaté lorsque les résonateurs capteurs 1-12, sont disposés soit dans un plan horizontal, soit dans un plan vertical.

Il y a une très nette variation d'intensité et une relative nuance, parfaitement perceptible en passant par exemple de ces positions initiales à la position moyenne donnant une inclinaison de 45°. Sans être nulles, les émissions ainsi recueillies, sont seulement toutes différentes et, de ce fait, peuvent également être utilisées pour des fonctions spécifiques bien particulières.

Toutes les inclinaisons intermédiaires, et toutes les combinaisons de circuits oscillants s'interpénètrent en se croisant, sont donc possibles et parfois même souhaitables pour certains emplois.

En se basant et en empruntant les techniques et les lois de l'électricité et de l'électromagnétisme, il a été possible de réaliser très facilement, ce qui est appelé dans ces techniques, un condensateur variable.

Véritable condensateur variable qu'il est possible d'obtenir réellement très facilement, grâce à un mécanisme approprié, qui oriente plus ou moins, par exemple du plan horizontal au plan vertical et, indépendamment les uns des autres, de façon à ce qu'ils s'interpénètrent progressivement, chaque élément d'émission d'effet de forme portant chacun un, ou, des circuits oscillants métalliques et, ou, polymétalliques.

Cet authentique condensateur variable est exactement ajustable pour obtenir une transformation en continue des émissions d'effets de forme, mais aussi, des rigoureuses et bien précises intensités et longueurs d'ondes émises par les résonateurs-capteurs-émetteurs 1-12.

Pour un rendement maximum de l'ensemble de la construction qui ne bénéficie pas d'un apport extérieur d'énergie électrique et ou électromagnétique, les résonateurs capteurs 1-12, doivent eux-mêmes être conçus pour devenir également des émetteurs d'émissions et d'effets de forme. Pour cela, il est essentiel suivant les buts recherchés et, ou, les intensités d'émissions souhaitées, que ces circuits d'émissions d'effets de formes soient calculés, structurés et, construits spécifiquement pour les différents objectifs désirés.

Pour les mêmes raisons, et pour obtenir les effets désirés, les géométries de forme qui, sur les diverses figures et par pure simplicité ont toujours été représentées par des circonférences et des volumes cylindriques, pourront très avantageusement, tant intérieurement qu'extérieurement, être de forme polygonale même quelconque , mais de préférence définie par des polygones réguliers.

De plus comme représenté sur les figures 12-13 et 14, les émissions des effets de forme, seront variables selon que les faces internes de ces résonateurs-capteurs-émetteurs comporteront des dépressions et/ou des protubérances linéaires en relief, courant partiellement ou en totalité sur le périmètre de ces structures.

Etant vues en coupe, ces dépressions pourront avoir comme forme de définition, toutes les figures géométriques connues, mais plus particulièrement, des demi-cercles, des demi-ellipses, des demi-polygones réguliers ou non et, ou, également être aussi définies par des portions de courbes issues de paraboles, de clothoides, de cycloides et épicycloides qui assurent des émissions d'effets de forme relativement caractéristiques.

Enfin, tout simplement, soit en alternance soit en totalité, pour augmenter ou simplifier les effets, par une classique surface plane de beaucoup moins grande efficacité, mais de très grande facilité de construction et de réalisation.

Il est parfois très profitable d'avoir recours à un montage alterné de ces éléments et, structures émettrices d'effets de forme, de façon à avoir des parfaites associations et combinaisons qui donneront un amalgame et un fusionnement harmonieux de l'ensemble.

Bien qu'il n'y ait aucun inconvénient à les disposer à un endroit quelconque, c'est très avantageusement aux foyers de ces diverses formes définissant les dépressions périphériques, que seront judicieusement et, profitablement disposés les circuits oscillants métalliques et, ou, polymétalliques.

De préférence, les circuits oscillants sont placés par rapport aux éléments massifs de façon à respecter les symétries de cers derniers, ils peuvent être mobiles autour de l'axe du canon émetteur auquel est associé le capteur résonateur dont il fait partie. Leurs mouvements peuvent être indépendants les uns des autres. Le mouvement, d'au moins certains d'entre eux, sont de sens opposés, ce qui produit dans certaines orientations une interpénétration des surfaces.

Les structures émettrices d'effet de forme peuvent avoir une section composée de deux arcs de cercle symétriques par rapport à l'axe du canon photonique émetteur d'électrons 13, 14 associés au capteur résonateur 1, 12 et se coupant sur celmui-ci en leur point le plus éloigné du corps creux principal 15.

Il a été constaté qu'un fonctionnement particulièrement harmonieux des résonateurs-capteurs-émetteurs 1-12, pouvait être obtenu en les surmontant tous, ou, en partie, d'un corps creux de résonance 49, homothétique du corps creux principal 15, qui recouvrira partiellement comme par exemple, représenté sur la figure 2, ou recouvrira entièrement les éléments d'émissions d'effets de forme supportant les circuits oscillants se trouvant dessous.

Bien qu'étant essentiellement préférés de forme pyramidale pour les syntonisations des cellules vivantes, ces corps creux secondaires, facilitant et augmentant le captage des résonateurs-capteurs-émetteurs 1-12, pourront être dans certains cas, et très avantageusement, remplacés par un, ou des volumes s'il y en a plusieurs, qui s'apparentent alors à ce qui a été dénommé précédemment : "PILE-EMETTRICE" qui seront rigoureusement et exactement conformes à toutes les descriptions qui ont déjà été données pour le corps creux de résonance principal 15.

Il y aura un plus grand intérêt, exactement comme pour le corps principal 15, a surmonter ces corps creux de résonance secondaire 49, d'un deuxième, voire d'un troisième corps toujours homothétique de celui qui le précède, cela toujours dans la même plage de rapports et, dans les mêmes orientations, avec également, l'emploi d'une part de miroirs convexes, plans ou concaves, et, d'autre part, de cristal pur pour le dernier de ces corps se trouvant à l'extrémité supérieure.

Selon un mode de réalisation préféré, les parois de ces corps secondaires 49, sont très avantageusement confectionnées à l'aide de multimatériaux comme les métaux magnétiques ou non, associées en panneaux sandwichs avec des matériaux organiques et, ou, minéraux diélectrique ou non, ainsi qu'en tout matériau connu, ou à venir, comme principalement, les matériaux de synthèse ou les matériaux composites.

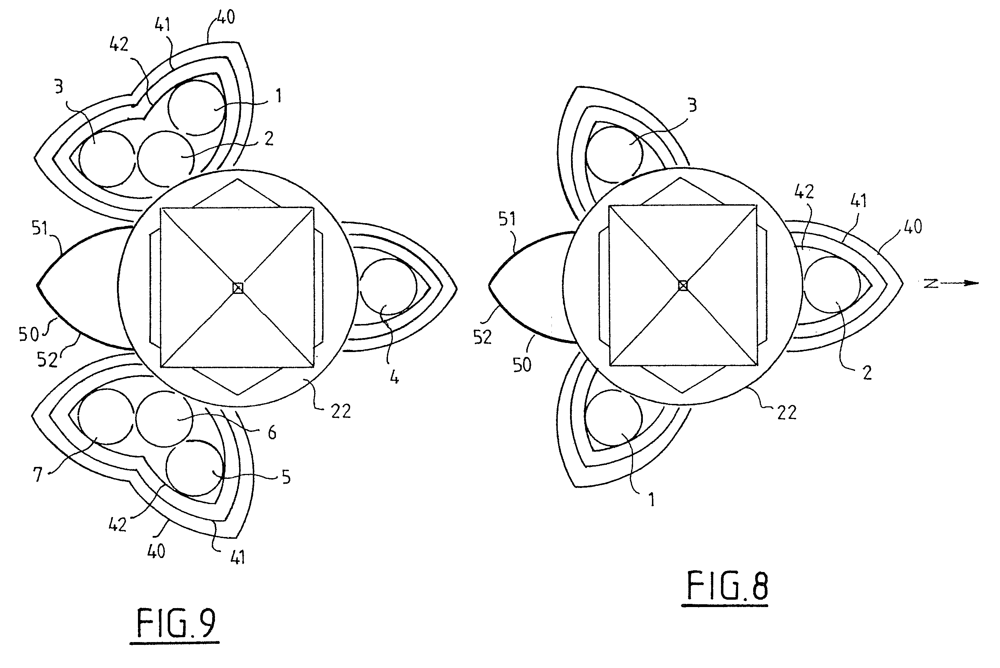

Il a été remarqué dans les expériences de recherches pour les mises aux points, que les influences primaires des résonateurs-capteurs-émetteurs 1-12, quels qu'ils soient, étaient renforcées quand ceux-ci employés à l'unité et, ou, associés en grappes comme représenté sur les figures 4 à 11, étaient enserrés comme symbolisé sur les figures 8-9 et 11, dans de nouvelles structures à éléments 40 à 43, émettant elles mêmes, des émissions d'effets de forme secondaires entièrement nouvelles et absolument très différentes, à cause entre autre, de la masse de ces éléments, mais surtout, de leurs ampleurs beaucoup plus importantes.

Comme pour les résonateurs-capteurs-émetteurs 1-12, ces nouveaux éléments 40 à 43 entrant dans la composition de ces nouvelles structures, pourront tant extérieurement qu'intérieurement, être de formes polygonales ouvertes, même quelconques, mais de préférence, définies par des portions ouvertes de polygones réguliers.

D'une manière préférée, la forme de ces nouveaux éléments, est composée de deux arcs de cercle et,ou, de deux portions de courbes issues de paraboles, de clothoides, de cycloides ou, d'épicycloides symétriques par rapport à l'axe du canon photonique émetteur d'électrons 13-14 associé au résonateur-capteur-émetteur, et se coupant sur celui-ci en leur point le plus éloigné des corps creux ou des résonateurs-capteurs-émetteurs quand ils sont associés en grappes comme représentés sur la figure 9.

Ces nouvelles structures à éléments 40 à 43, bénéficieront de toutes les techniques de formes, de matériaux, d'orientation et de construction déjà décrites pour le corps principal 15, la base 22 ainsi que pour les résonateurs-capteurs-émetteurs I-2, et, plus particulièrement de ceux-ci, les éléments 40 à 43, hériteront des dépressions et des protubérances en relief courant partiellement ou en totalité sur leurs périmètres.

En dehors de la représentation des coupes des circuits oscillants et de leurs éléments supports dans les résonateurs-capteurs-émetteurs 1-2, les figures 12-13 et 14 représentent en plus, les coupes des éléments 40 à 43 de ces nouvelles structures d'émissions d'effets ae forme.

Il est souhaitable de disposer aussi à cause de leurs ampleurs et de leurs masses très considérables des circuits oscillants métalliques et, ou, polymétalliques, de bien plus grandes dimensions, qui donneront de ce fait, une nouvelle gamme d'ondes de longueurs beaucoup plus importantes que celles émisent par les résonateurs-capteurs-émetteurs I-2.

Selon un mode de réalisation préféré, les canons photoniques émetteurs d'électrons 13-14, entrant dans la constitution des générateurs d'ondes conformément à l'invention, comportent chacun et comme représenté sur la figure 2 :

. Premièrement, un condensateur accélérateur amplificateur linéaire 44, dont l'entrée d'admission du faisceau d'ondes débité par le résonateur capteur émetteur I, se trouve juste en regard des ouvertures des éléments émetteurs d'émissions d'effets de forme, supportant eux-mêmes les circuits oscillants 25 à 27 dont les éclateurs 34 à 39 se trouvent également exactement au même endroit.

. Deuxièmement, une lentille optique convergente 45, de faible grossissement, mais de très grand diamètre.

. Troisièmement, un condensateur accélérateur amplificateur concentrateur 46.

. Quatrièmement, le point focal 47, de la lentille optique convergente qui est utilisé pour obtenir juste à cet endroit bien précis, et, grâce aux éclateurs de circuits oscillants métalliques et, ou, polymétalliques de très petites dimensions, des ondes supplémentaires de très hautes fréquences.

. Cinquièmement, un condensateur accélérateur amplificateur diffuseur 48, qui, grâce à sa forme diffusante, élargi notablement le faisceau d'ondes qui a été au préalable singulièrement concentré au point focal 47, par la lentille optique convergente 45, pour qu'il couvre entièrement le miroir récupérateur 20, qui est employé plus spécifiquement pour les générateurs d'ondes devant syntoniser les cellules humaines vivantes.

. Sixièmement, pour les générateurs employés plus spécifiquement en biologie humaine, un miroir récupérateur, diviseur, répartiteur directionnel 20, qui est chargé premièrement de renvoyer sous le bon angle, le faisceau d'ondes récupéré à la sortie du canon émetteur, mais deuxièmement, de le répartir aussi en le divisant afin que sa forme circulaire d'émission d'origine, soit ordonnée pour couvrir dans sa totalité, la plage rectangulaire que représente par exemple un corps humain 16, placé sur la stèle 17. Les miroirs plans 18 et 19, n'ayant uniquement comme fonction, que de renvoyer ces faisceaux d'ondes sur le patient 16.

Les flèches mises dans l'axe des canons photoniques émetteurs d'électrons sur les figures 2 et 3, font d'une part clairement apparaître le fonctionnement et, d'autre part, ,matérialisent parfaitement le sens et le trajet des faisceaux d'ondes.

Pour des canons photoniques émetteurs d'électrons devant être obligatoirement plus courts en longueur à cause d'impératifs de construction, il peut être réalisé une structure plus courte et légèrement simplifiée conformément à la figure 3, dans laquelle le corps creux n'a pas été représenté, et ou le condensateur accélérateur amplificateur linéaire 44 a été supprimé.

La lentille optique convergente 45, qui se trouve alors dans cette circonstance, directement en regard des ouvertures des éléments émetteurs d'émissions d'effets de forme ainsi que des éclateurs 34 à 39, devra en l'occurence, être de préférence d'un plus grand diamètre, tout en étant associée plutôt à des éléments d'émissions d'effets de forme de hauteurs différentes comme schématisé sur la figure 13, et dont le centre de concentration qui peut être aussi appelé le centre de gravité figuré, se trouve si possible, en concordance avec l'axe directeur des canons photoniques émetteurs d'électrons 13-14.

Par souci de simplification, les canons photoniques émetteurs d'électrons 13-14, peuvent être constitués d'un simple tube cylindrique. Mais il y a de grands avantages à choisir selon les besoins et les buts recherchés, premièrement des tubes unitairement coniques et, ou, emboîtés successivement les uns derrière les autres, soit comme pour ce qui est désigné par le vocable : "PILES EMETTRICES", par plusieurs tronçons de cônes, assemblés d'une façon opposée par exemple par les grands diamètres se regardant, puis ensuite par les petits diamètres et ainsi de suite. Deuxièmement, avec des sections pour ces tubes cylindriques et, ou coniques,plus complexes que le cercle.

Ces sections pourront avantageusement être définies par des géométries quelconques, ou définies par des polygones réguliers ou encore issues de toutes les courbes régulières connues et engendrées mathématiquement.

Les matériaux de ces tubes peuvent être de toutes matières connues et, ou à venir, mais il a été observé d'excellents résultats avec des tubes métalliques, des tubes en matières de synthèse, et même , avec de simples tuyaux en ciment ou en amiante-ciment.

Les condensateurs accélérateurs amplificateurs, qu'ils soient linéaires, concentrateurs ou diffuseurs, peuvent être constitués en partie ou en totalité, ou encore en alternance et ou en interpénétration des divers éléments s'engageant plus ou moins les uns dans les autres, avec un pas modulaire constant ou progessif, mais qui également peut être multiple, par des solénoides, qui, dans le domaine des émissins d'effets de forme, jouent parfaitement les rôles demandés et recherchés.

Pour les mêmes raisons, ils peuvent être aussi réalisés grâce à des empilements de disques régulièrement espacés, mais non limitativement, comme dans un condensateur électrique.

Ces empilements de disques, ayant alternativement des évidements centraux pouvant avoir toutes les formes connues, mais, de surfaces inégales afin de créer une différence de potentialité de surface, mais aussi de masse, d'échange et de périmètre, pour jouer comme dans certains appareils de mesure électrique, sur les influences des effets de bords et de pointes ; ce qui permet également à ces endroits d'obtenir des effets Corona qui donnent une bien meilleure ionisation de l'air.

Comme en émissions d'effets de forme la nature des matériaux entrant dans les constructions, n'intervient que partiellement , ces disques peuvent être en tous matériaux connus et ou, à venir. Cependant, d'extraordinaires résultats ont été observés avec des disques en métaux multiples ou non.

Les solénoides cylindriques sont eux aussi d'une façon identique et, pour les mêmes raisons, élaborés à partir de fils, tiges ou barres de tous ces matériaux en étant pour leurs constructions, à pas d'hélice à sens horaire ou anti-horaire constant, ou, plus avantageusement à pas d'hélice progressif ou, multiples sur la longueur.

Ces solénoides peuvent également coniques, ou bi-conique simple, ou encore, bi-coniques multiples.

Toutes ces variations de pas d'hélice d'une part, et de diamètre de spires d'autre part, permettent d'obtenir par leurs combinaisons des ondes entretenues modulées tant en amplitudes qu'en fréquences.

De même, les combinaisons concentriques des solénoides et des empilements de disques dans un même condensateur accélérateur amplificateur, sont particulièrement recommandées.

Le premier montage prévoyant le solénoide en périphérie avec l'empilage des disques au centre. Le deuxième montage plus spécialement préconisé, dispose le solénoide au centre, dans les évidements des disques.

Mais toutes combinaisons par interpénétrations totales ou partielles de certains disques avec certaines spires des solénoides sont possibles, augmentant ainsi les éventualités à l'infini.

Un montage original et fort intéressant consiste d'ailleurs à monter conjointement, soit en interpénétration, soit concentriquement, deux ou plusieurs enroulements cylindriques, coniques ou multiformes, l'un enserrant et, ou interpénétrant l'autre, avec des sens d'enroulement inversés pour ces solénoides.

Il a été remarqué de plus, qu'il y avait parfois utilité pour certains générateurs d'ondes de tailles bien particulières, a remplacer le condensateur accélérateur amplificateur linéaire 44, par un condensateur accélérateur amplificateur concentrateur 46 qui, dans ce cas, aura son plus petit diamètre de spires et, ou, de disques, disposé en regard de la lentille optique convergente 45.

Bien qu'il soit relativement facile de disposer tout au long des canons photoniques émetteurs d'électrons 13-14, des circuits oscillants métalliques et, ou polymétalliques de petites dimensions, dont les éclateurs disposés dans l'axe de ceux-ci donneront mathématiquement des longueurs d'ondes faibles avec fréquences relativement élevées, il est préférable, pour obtenir de très petites longueurs d'ondes et des très hautes fréquences absolument et impérativement indispensables pour acquérir par exemple et, entre autre, les mêmes effets thérapeutiques que les appareils existant déjà mais fonctionnant uniquement avec un apport d'énergie électrique qui, comme déjà expliqué, est plus ou moins relativement néfaste, l'invention a prévu pour obtenir ces très petites longueurs d'ondes et ces très hautes fréquences, qui sont essentiellement vitales, de disposer au point focal 47 de la lentille optique convergente 45, les éclateurs de toute une série de très petits circuits oscillants métalliques et, ou polymétalliques dont les diamètres seront même parfois minuscules pour certains générateurs d'ondes de très petites tailles.

Etonnamment, il est alors ainsi obtenu dans les générateurs d'ondes conformes à l'invention, trois extraordinaires plages de fréquences différentes, fournies par les trois remarquables plages de longueurs d'ondes bien distinctes l'une de l'autre, et qui peuvent être définies sans aucune référence aux longueurs d'ondes et aux fréquences connues par exemple des radiations lumineuses, de l'électricité ou , hertziennes etc... mais, uniquement pour, et en rapport avec les générateurs d'ondes de l'invention en plage de grandes ondes à basses fréquences dispensées par les éléments 40 à 43, enserrant les résonateurs capteurs émetteurs 1-2, qui procurent eux-mêmes, la plage des longueurs d'ondes intermédiaires à fréquences moyennes, et enfin, la plage des petites ondes à hautes fréquences dispensées par les micros circuits oscillants disposés au point focal 47 de la lentille 45.

Ces trois exceptionnelles plages de longueurs d'ondes et de fréquences très différentes, couvrent en totalité les demandes et les besoins bien distincts et très particuliers, et contribuent à des syntonisations absolument parfaites de tous les cas de figures rencontrées.

Selon un mode de réalisation préféré, il a été constaté qu'un revêtement réfléchissant tel que des miroirs et, ou plus simplement, des films appropriés en matériaux de synthèses ayant les mêmes propriétés, disposés sur les corps 15-23-49 et aussi si besoin, sur les éléments des montages dénommés, "PILES EMETTRICES", permet d'éliminer très efficacement certaines perturbations de fonctionnement correct de l'ensemble, qui est due à quelques ondes atmosphériques et, ou cosmiques toujours nettement décelables, mais encore très souvent inconnues qui, grâce à ces revêtements réfléchissants, sont totalement renvoyés pour se perdre au loin.

D'une manière analogue, il a également été constaté que des perturbations engendrées par certaines émanations telluriques dissonantes, pouvaient être entièrement supprimées en noyant dans la dalle servant de fonction à l'ensemble d'un générateur d'ondes conforme à l'invention, un simple grillage métallique de la même superficie, ou des fils entrecroisés, qu'il est avantageux de choisir en cuivre à cause de leurs plus grandes conductivités, reliés à un, ou des diffuseurs au loin, et communément appelés prises de terre disposées au Nord magnétique.

Il est apparu que certains générateurs d'ondes, principalement ceux réalisés avec des corps creux 15 et des corps secondaires de résonance 49 constitués de forme pyramidales, pouvaient parfois et, dans certaines circonstances, émettre à l'extérieur et au sud magnétique, un faisceau d'ondes très nettement perceptible qui a été découvert en 1937.

Ce faisceau d'ondes toujours décelable, groupant un certain nombre d'ondes toujours non identifiées, est toujours, jusqu'à ce jour, uniquement désigné que par le vocable de : "Vert-Négatif", à cause de sa situation qui le place dans le spectre des couleurs visibles à l'opposé du vert.

Les rayonnements de ce "Vert-Négatif" pouvant à la longue et dans certaines situations, être relativement gênants à l'environnement, l'invention à découvert qu'ils pouvaient être totalement éliminés par l'adjonction d'un enclos d'absorption 50, délimité par deux éléments de forme 51 et 52, disposés au Sud magnétique, et se coupant sur l'axe Nord-Sud passant par le centre du corps creux 15 et parfois si nécessaire, des corps secondaires de résonance 49.

Ces deux éléments de forme 51 et 52, bien que n'étant pas capteurs et émetteurs d'émissions d'effets de forme, mais à cause, et, suivant les tailles, les masses, les implantations, les matériaux, les dispositions intérieures et extérieures etc, devront être réalisés chaque fois en bénéficiant eux aussi, de toutes les techniques de formes, de matériaux, et de construction déjà décrites pour les éléments d'émissions d'effets de formes des résonateurs capteurs émetteurs 1-12 ainsi que des structures à éléments 40 à 43.

Pour les générateurs d'ondes conformes à l'invention qui doivent s'appliquer à syntoniser les cellules vivantes, la stèle 17, qui pourra se situer en tout point du corps creux principal 15, et plus particulièrement à toutes les hauteurs accessibles sur l'axe vertical passant à l'aplomb des corps homothétiques 23 et 24, supporte un siège qui prend toutes les positions possibles de repos agréable.

Ce siège pour faciliter une syntonisation parfaitement harmonieuse, a la particularité de comporter sous son revêtement premièrement une importante nappe de fils métalliques et, ou polymétalliques tressés et très souple, qui épouse agréablement les formes de corps, et qui avec les barres et les amas de mêmes métaux disposés au dessous d'elle, joue le rôle de tampon amortisseur, et aussi d'égalisateur, équilibrant et régularisant grâce à leurs importantes masses et ampleurs, les flux des faisceaux d'ondes venant des divers canons émetteurs périphériques.

Deuxièmement, pour les mêmes raisons évoquées ci-dessus, une multitude de petites pastilles magnétiques de faible puissance, insérées dans la nappe métallique tressée et répartie sur la totalité de sa superficie plus, en des points judicieusement étudiés et choisis en conséquence, de gros aimants de fortes et, très fortes puissances. Dans les générateurs industriels, tous ces aimants seront disposés au point de convergence commun de tous les axes des canons émetteurs.

Des effets particulièrement bénéfiques sont obtenus lorsque les générateurs d'ondes de l'invention comportent plusieurs résonateurs-capteurs-émetteurs 1 à 12, et que ceux-ci sont régulièrement disposés autour du corps creux principal 15.

Une disposition particulièrement intéressante est obtenue avec trois structures émettrices de forme dont les axes de symétrie se coupent au centre du corps creux principal et sont régulièrement répartis autour de celui-ci. L'un de ces axes est orienté au Nord magnétique et de manière symétrique certaines d'entre elles,peuvent entourer plusieurs capteurs émetteurs. Une telle réalisation est représentée sur les figures 8 et 9.

Les figures 8 et 9, proposent différentes positions qui peuvent être adoptées pour placer les résonateurs-capteurs-émetteurs par rapport au corps creux principal pour des générateurs de petites puissances,et aussi, pour des syntonisations de cellules vivantes.

Pour ces appareils, il est particulièrement harmonieux d'associer plusieurs résonateurs-capteurs-émetteurs 1 à 12 à un même canon photonique émetteur d'électrons 13-14, à condition que ceux-ci, soient régulièrement orientés par rapport à l'axe du canon photonique émetteur d'électrons 13-14.

Dans ces générateurs d'ondes, les trois plages de longueurs d'ondes, et les trois plages de fréquences, sont réellement dispensées grâce à tous les éléments inventés, et qui doivent constituer des générateurs parfaits.

Pour les générateurs d'ondes devant dynamiser spécifiquement les cellules humaines par syntonisation, les canons photoniques émetteurs d'électrons 13, 14 ont avantageusement leurs axes parallèles au sol.

Et comme déjà expliqué, grâce aux jeux de miroirs 18, 21 judicieusement disposés, les faisceaux d'ondes émis sont divisés, redressés puis répartis sur la personne qui repose confortablement sur la stèle 17. Par contre, pour les générateurs d'ondes d'emplois industriels, il n'y a plus l'impérative nécessité de diriger obligatoirement les faisceaux d'ondes de façon à ce qu'ils tombent sensiblement sous un angle de 30° environ, de part et d'autre de la personne à dynamiser et syntoniser. De ce fait, les générateurs d'ondes industriels ont tous ou, en partie, les axes de leurs canons émetteurs disposés suivant les génératrices d'un cône, dont la base repose au sol. Cela de façon à ce que tous les axes des canons émetteurs, aient un point de convergence commun, qui se trouve exactement au niveau du support ou doit s'opérer la parfaite dynamisation et, l'irréprochable syntonisation.

Les figures 4 et 5, représentent des générateurs simplifiés, où les grandes longueurs d'ondes et les basses fréquences, ne sont absolument pas indispensables. Ces générateurs sont en général employés pour des traitements industriels et à petites échelles, de produits constitués de matière dite "inerte".

Selon cette réalisation, le générateur d'ondes ne comporte pas de structures émettrices de forme autour des capteurs émetteurs.

Industriellement, il est parfois absolument indispensable de disposer de générateurs d'ondes de très grandes puissances, impérativement nécessaires pour des traitements industriels en continu, comme par exemple, dans les industries des boissons et des conserves de toutes sortes, des eaux d'emplois usiniers etc...

En effet, industriellement, les passages sous les générateurs d'ondes conformes à l'invention, des liquides et, ou des produits doivent être obligatoirement de très courtes durées.

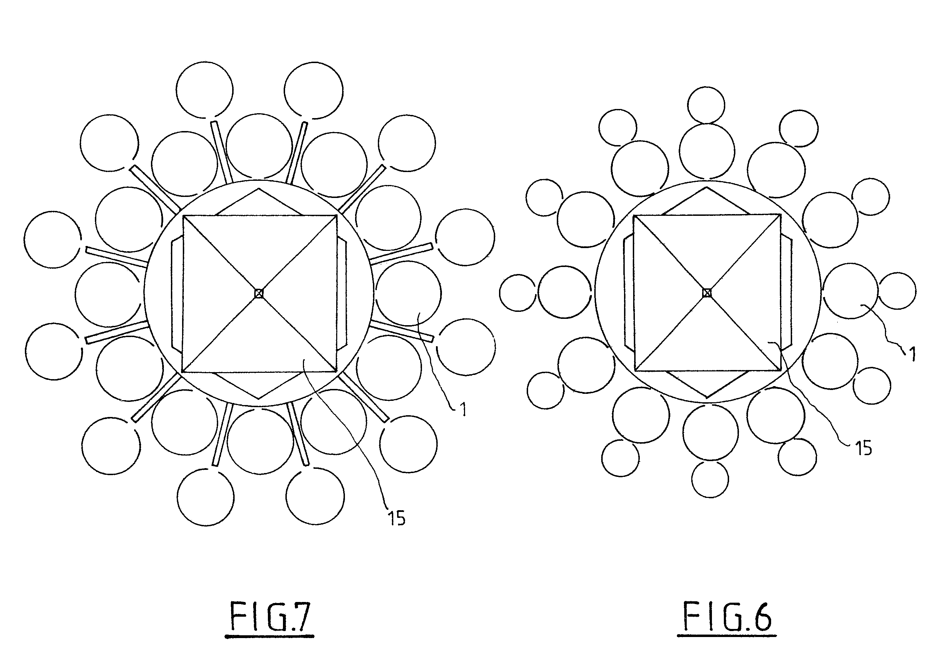

A cause de cette réduction du temps d'exposition, il est donc impératif d'augmenter d'une façon considérable, les puissances des générateurs. Cette prodigieuse augmentation de puissance, ne peut alors être obtenue qu'avec la multiplication des résonateurs-capteurs-émetteurs comme représenté sur les figures 6 et 7, où il n'y a plus de structure émettrice de forme 40 à 43, qui ne sont, en général plus indispensables comme précédemment expliqué, mais ou la puissance au point de convergence des vingt quatre canons émetteurs du générateur représenté sur la figure 7, est telle, que le passage en continu des liquides et, ou des produits à des rythmes industriels est parfaitement assuré.

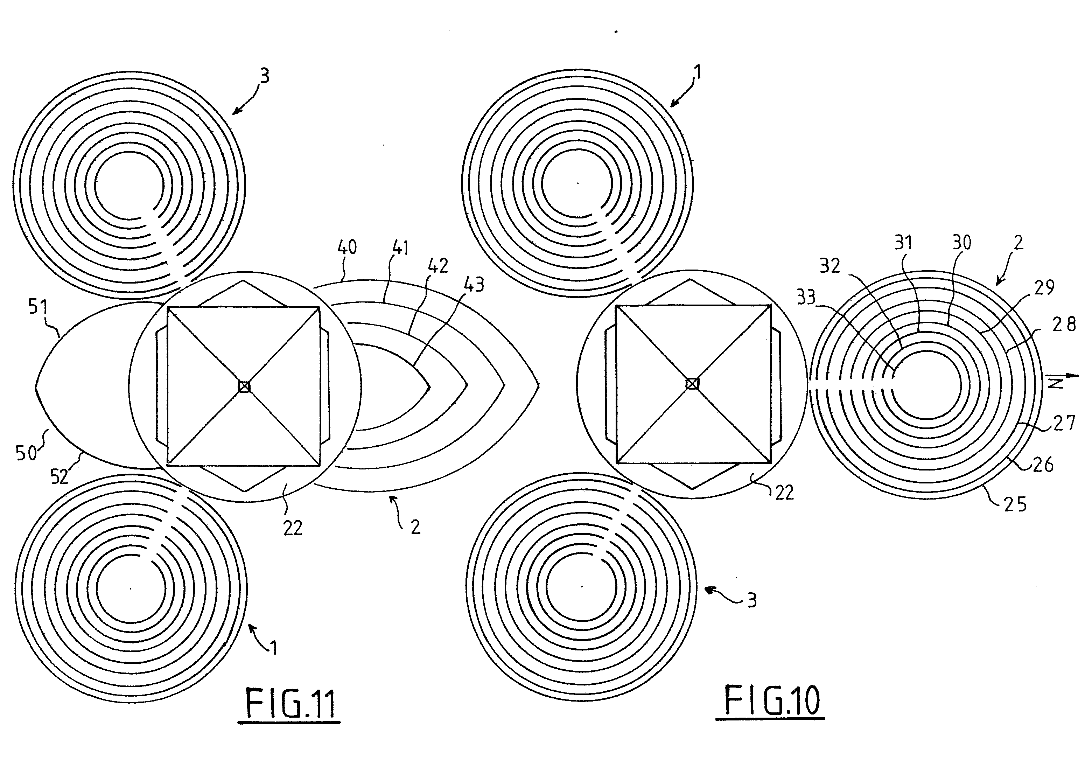

Néanmoins, certaines applications industrielles ayant nécessairement besoin de basses fréquences, découlant de grandes longueurs d'ondes, l'invention a étudié avec les figures 10 et 11, des générateurs d'ondes toujours conformes à l'invention , ayant des résonateurs-capteurs-émetteurs de très grandes dimensions qui réalisent ce qui est souhaité, réclamé et demandé par les industries.

Industriellement,des générateurs d'ondes conformes à l'invention, pouvant par exemple, pour l'agriculture, l'arboriculture, l'horticulture, débiter en continu d'énormes quantités d'eau dynamisée et magnétisée, chargé de plus, des oligo-éléments métalliques essentiels, permettent l'irrigation d'immenses plantations.

Tous les éléments métalliques existent dans les plantes à l'état d'oligo-éléments et servent de régulateurs de croissance. Sous l'action de telles irrigations, la fertilité des plantes augmente, leurs tissus rajeunissent et ils se protègent mieux contre les gelées. Ces plantes sont également plus vertes que les autres, et ont parfois une croissance et une vitalité prodigieuse par rapport à d'autres servant de témoins.

Les travaux de recherches ont démontré que l'eau d'arrosage dynamisée et magnétisée sous des générateurs d'ondes conformes à l'invention, a des effets actifs, non seulement sur l'absorption de cette eau et des sels nutritifs qu'elle contient, mais aussi, sur d'autres processus vitaux, y compris la division des cellules et la rapidité des germinations avec un taux de réussite toujours nettement supérieur à tous les autres lots servant de témoins. Le mûrissement des fruits et des tomates par exemple, est toujours dans chaque cas de figure, très nettement plus rapide.

Les emplois possibles dans l'industrie sont immenses et ne peuvent être ici tous évoqués et énumérés. Toutefois, il sera donné ci-dessous deux exemples d'utilisation choisis parce que parmi les plus étonnants.

En effet, il a été constaté d'une façon surprenante, qu'une eau ordinaire traitée industriellement par un générateur d'ondes conforme à l'invention d'une grande puissance, devenait dynamisée et aussi magnétisée, et, réduisait d'une façon très appréciable les poussières de forage des trous de mines améliorant ainsi la condition des mineurs.

D'une façon aussi surprenante, il a été également constaté entre autres, que l'emploi de la même eau, augmentait notablement la cohésion du béton et, par conséquent, sa résistance. Les bétons légers deviennent avec cette eau, presque deux fois plus solides et plus durs que les bétons ayant été réalisé avec une eau ordinaire. De plus, ces bétons sèchent plus rapidement et sont plus imperméables à l'eau.

A ce point de la description de l'invention, son importance et son efficacité sont clairement apparentes. Les traitements qu'elle permet, tant biologiquement qu'industriellement, peuvent être harmonieusement associés à la magnétothérapie, l'aimantothérapie, ainsi que la chromothérapie. Tous les moyens nécessaires à la mise en oeuvre de ces méthodes tels que des aimants, des écrans permettant de projeter des images colorées peuvent être incorporés au corps creux de façon à augmenter de son efficacité l'ensemble des appareils conformes à l'invention, et de ce fait, font partie intégrante de l'invention.

## Revendications

1) Générateur d'ondes caractérisé en ce qu'il comporte :
- au moins un résonateur capteur (1-12),
- au moins un canon photonique émetteur d'électrons (13, 14) associé à un ou plusieurs résonateurs capteurs (1-12) et amplifiant les ondes,
- un corps creux principal (15) destiné à syntoniser les ondes avec un corps (16) placé sensiblement en son milieu sur un support ou une stèle (17) vers lequel sont dirigées les ondes sortant des canons émetteurs.

2) Générateur d'ondes selon la revendication 1, caractérisé en ce qu'il comporte des structures émettrices d'effets de formes (40-43).

3) Générateur d'ondes selon l'une quelconque des revendications 1 et 2, caractérisé en ce qu'il comporte des corps creux surmontant certains résonateurs capteurs ou certaines structures émettrices de formes.

4) Générateur d'ondes selon l'une quelconque des revendications 1 à 3, caractérisé en ce que certains corps creux sont pyramidaux.

5) Générateur d'ondes selon l'une quelconque des revendications 1 à 4, caractérisé en ce que certains corps creux ont une forme de cône à directrice ellipsoïdale.

6) Générateur d'ondes selon l'une quelconque des revendications 1 à 14, caractérisé en ce que la surface de certains corps creux est constituée de plusieurs couches de matériaux différents ou identiques.

7) Générateur d'ondes selon l'une quelconque des revendications 1 à 6, caractérisé en ce que l'un au moins des corps creux est surmonté d'un deuxième corps homothétique du premier dans un rapport approximatif de 1 à 10.

8) Générateur d'ondes selon la revendication 7, caractérisé en ce que la face inférieure du deuxième corps creux porte un miroir.

9) Générateur d'ondes selon l'une quelconque des revendications 7 et 8, caractérisé en ce que l'un au moins du deuxième corps est surmonté d'un troisième corps homothétique du second dans un rapport approximatif de 1 à 10.

10) Générateur d'ondes selon la revendication 9, caractérisé en ce que le troisième corps est un cristal.

11) Générateur d'ondes selon l'une quelconque des revendications 1 à 10, caractérisé en ce que les résonateurs capteurs comprennent des circuits oscillants (25 à 35), chacun comprenant des circuits, conducteurs plans quasifermés, terminés à leurs extrémités par des éclateurs (34 à 39).

12) Générateur d'ondes selon la revendication 11, caractérisé en ce que les circuits conducteurs sont constitués d'une pluralité de métaux chacun d'entre eux étant constitué par un seul de ces métaux.

13) Générateur d'ondes selon l'une quelconque des revendications 11 et 12, caractérisé en ce que plusieurs circuits oscillants d'un même résonateur capteur sont mobiles et que le mouvement, d'au moins certains d'entre eux, sont de sens opposés, ce qui produit dans certaines orientations une interpénétration des surfaces.

14) Générateur d'ondes selon l'une quelconque des revendications 1 à 13, caractérisé en ce que le ou les canons photoniques émetteurs d'électrons 13, 14 comportent chacun un condensateur-amplificateur-concentrateur focalisant les ondes, un ensemble de circuits oscillants métalliques plans dont les éclateurs sont situés au voisinage du point de focalisation des ondes, un condensateur amplificateur diffuseur (47) dirigeant les ondes amplifiées vers le support ou la stèle contenue dans le corps principal.

15) Générateur d'ondes selon la revendication 14, caractérisé en ce que le canon photonique émetteur d'électrons comporte une lentille optique.

16) Générateur d'ondes selon l'une quelconque des revendications 14 à 15, caractérisé en ce que les condensateurs accélérateurs comportent des surfaces de révolution d'axes confondus avec celui des condensateurs accélérateurs et comportant des évidements centraux pouvant avoir des formes d'étoile et susceptibles d'engendrer des effets de forme et des effets de pointe Corona.

17) Générateur d'ondes selon l'une quelconque des revendications 1 à 16, caractérisé en ce qu'il comporte plusieurs capteurs résonateurs associés à un même canon photonique émetteur d'électrons et régulièrement orienté par rapport à l'axe de ce dernier.

FIG.1

EP 0 312 452 A1

FIG.2

FIG.3

FIG.5

FIG.4

EP 0 312 452 A1

FIG.7

FIG.6

EP 0 312 452 A1

FIG.9

FIG.8

EP 0 312 452 A1

FIG.10

FIG.11

FIG.12

FIG.13

FIG.14

FIG.15

EP 0 312 452 A1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | FR-A-2 264 406  (FANTUZZI) <br> * En entier * <br> --- | 1-5,14 | A 61 N   1/16 <br> H 05 F   7/00 <br> A 23 L   3/26 <br> C 12 H   1/16 |
| A | FR-A-2 506 533  (CORNILLON) <br> * En entier * <br> --- | 1-4,11, 16 | |
| A | FR-A-2 592 770  (CORNETTE) <br> * Page 1, ligne 23 - page 2, ligne 34; <br> page 4, ligne 5 - page 6, ligne 14; <br> figures * <br> --- | 1,2,4 | |
| A | FR-A-2 236 521  (CARTILLIER) <br> * Page 2, ligne 36 - page 5, ligne 40; <br> figures * <br> --- | 11,12 | |
| A | GB-A-  385 987  (STETTNER) <br> * En entier * <br> --- | 1,3,4 | |
| A | LU-A-   63 265  (OLATOKUNBO OKIKIOLU) <br> * En entier * <br> --- | 11 | |
| A | FR-A-2 554 354  (DUPIN) <br> * Page 2, ligne 21 - page 4, ligne 16 * <br> --- | 7,9 | DOMAINES TECHNIQUES RECHERCHES (Int. Cl.4) <br><br> A 61 N <br> H 05 F <br> A 23 B <br> A 23 L <br> C 12 H |
| A | DE-A-3 543 765  (KARREMAN) <br> * En entier * <br> --- | 1 | |
| A | DE-A-3 525 521  (KAUSCH) <br> * En entier * <br> ----- | 1 | |

Le présent rapport a été établi pour toutes les revendications

| Lieu de la recherche | Date d'achèvement de la recherche | Examinateur |
|---|---|---|
| LA HAYE | 13-12-1988 | LEMERCIER D.L.L. |